# EUROPEAN PATENT SPECIFICATION

(11) **EP 2 013 624 B1**
(45) Date of publication and mention of the grant of the patent: **03.08.2011**
(21) Application number: 07733988.5
(22) Date of filing: 14.03.2007
(51) Int. Cl.: G01N 33/68, C07H 7/027, C07H 15/04, G01R 33/465

(54) **METHOD FOR THE IDENTIFICATION OF NEW LEADS FOR DRUG CANDIDATES**
VERFAHREN ZUR IDENTIFIZIERUNG NEUER LEITVERBINDUNGEN FÜR ARZNEISTOFFKANDIDATEN
PROCÉDÉ D'IDENTIFICATION DE NOUVELLES TÊTES DE SÉRIE POUR DES MÉDICAMENTS CANDIDATS

(30) Priority: 14.03.2006 US 782104 P
(43) Date of publication of application: 14.01.2009
(73) Proprietor: Universität Basel, 4003 Basel (CH)
(72) Inventor: ERNST, Beat, CH-4312 Magden (CH); CUTTING, Brian, CH-4125 Riehen (CH); SHELKE, Sachin V., CH-4055 Basel (CH)
(86) International application number: PCT/IB2007/000643
(87) International publication number: WO 2007/105094

(56) References cited:
- WO-A-2006/116736
- WO-A2-03/000709
- CONGREVE M S ET AL: "Detection of ligands from a dynamic combinatorial library by X-ray crystallography" ANGEWANDTE CHEMIE. INTERNATIONAL EDITION, WILEY VCH VERLAG, WEINHEIM, DE, vol. 42, no. 37, 29 September 2003 (2003-09-29), pages 4479-4482, XP002275867 ISSN: 1433-7851
- LEWIS W G ET AL: "Click chemistry in situ: acetylcholinesterase as a reaction vessel for the selective assembly of a femtomolar inhibitor from an array of building blocks" ANGEWANDTE CHEMIE. INTERNATIONAL EDITION, WILEY VCH VERLAG, WEINHEIM, DE, vol. 41, no. 6, 2002, pages 1053-1057, XP002967538 ISSN: 1433-7851
- JAHNKE WOLFGANG ET AL: "Second-site NMR screening and linker design" CURRENT TOPICS IN MEDICINAL CHEMISTRY, BENTHAM SCIENCE PUBLISHERS, HILVERSUM, NL, vol. 3, no. 1, 2003, pages 69-80, XP008080781 ISSN: 1568-0266
- REES DAVID C ET AL: "FRAGMENT-BASED LEAD DISCOVERY" NATURE REVIEWS. DRUG DISCOVERY, NATURE PUBLISHING GROUP, BASINGSTOKE, GB, vol. 3, no. 8, August 2004 (2004-08), pages 660-672, XP009072515 ISSN: 1474-1784
- SHELKE SACHIN V ET AL: "Synthesis of sialic acid derivatives as ligands for the myelin-associated glycoprotein (MAG)." BIOORGANIC & MEDICINAL CHEMISTRY 15 JUL 2007, vol. 15, no. 14, 15 July 2007 (2007-07-15), pages 4951-4965, XP002440433 ISSN: 0968-0896

## Description

### FIELD OF THE INVENTION

The present invention is directed at a combinatorial approach for identifying high affinity ligands or new leads for drug candidates. The target may be unknown and/or may include one or more unknown binding sites. The present disclosure is also directed at kits for the method described herein.

### BACKGROUND

Next to high-throughput screening (HTS), fragment-based drug design or fragment based screening (FBS) has established itself as a powerful tool for drug discovery. In FBS small chemical structures ("fragments") are identified that often only exhibit weak binding affinity to a target (Rees, 2004). The concept was introduced as early as 1981 by Jencks (Jencks, 1981). At the time, Jencks's concept did not have an immediate impact on drug design, since two principal problems remained: (a) identifying suitable fragments that bind a target in close proximity to each other and (b) linking the fragments without distorting the binding of the individual fragments. Only in 1996 with the introduction of a method called SAR-by-NMR (SAR="structure activity relationship"), fragment-based drug design took its course. SAR-by-NMR relies on the combination of serendipity screening and knowledge of the three dimensional structure of the target (WO97/18471; WO97/18469; US patent 5,804,390; US patent 5,698,401).

The term target-guided synthesis (TGS) is used to describe the fact that a target is involved in the modular build up of molecules. Generally, the goal is that the so assembled molecules have a higher binding affinity to the target than their individual parts (here" fragments") and are able to exert a desired effect on the target. Sharpless and Kolb developed a strategy which relies on irreversible TGS to produce high affinity inhibitors from small fragments. They worked with acetylcholine esterase (AChE), a key player in neurotransmitter hydrolysis in the central and peripheral nervous system. Using their knowledge of the structure of the active site of AChE, fragments were designed to bind either the peripheral anionic site or the active center. The fragments carried azide and acetylene groups at the end of flexible spacers. While 52 fragment combinations were possible, the AChE "selected" four pairs of fragments that reacted via the azide and acetylene groups thus forming composite compounds.

These compounds were more potent than any other non-covalent organic AChE inhibitor and had dissociation constants (Kd) of up to 99fM. Sharpless and Kolb's approach could even be used for the discovery of inhibitors from reagents that were not previously known to interact with AChE peripheral sites. (Röper & Kolb, 2006).

Sharpless and Kolb worked with a well defined target which allowed a rational design of a fragment pool for testing. A patent application (WO2006/116736 directed to the principle of synthesizing imaging probes by click chemistry was filed. Second-site NMR screening and linker design was described by Jahnke et al. (Jahnke, 2000, Jahnke, 2003). The synthesis of sialic acid derivatives as ligands for the myelin-associated glycoprotein (MAG) was published in 2007 (Shelke, 2007).

Lewis et al. (Lewis, 2002) reported the reaction of tacrine carrying a linker and an azide function with phenanthridine carrying a linker and an acetylene group in the presence of acetylcholinesterase (AChE) to give new strongly binding AChE ligands by click reaction. Congreve et al. (Congreve, 2003) described the detection of ligands for cyclin-dependent kinase 2 (CDK2) from a "dynamic" combinatorial library of substituted phenylhydrazines and substituted oxindoles by soaking CDK2 crystals with library components and detection of binding by X-ray crystallography.

There remains a need for designing drug lead molecules (leads for drug candidates) for less well defined targets.

The publications and other materials, are including patents, used herein to illustrate the invention and, in particular, to provide additional details with respect to the practice. For convenience, the publications are referenced in the following text by author and year and are listed in the appended bibliography in alphabetical order.

### BRIEF DESCRIPTION OF THE FIGURES

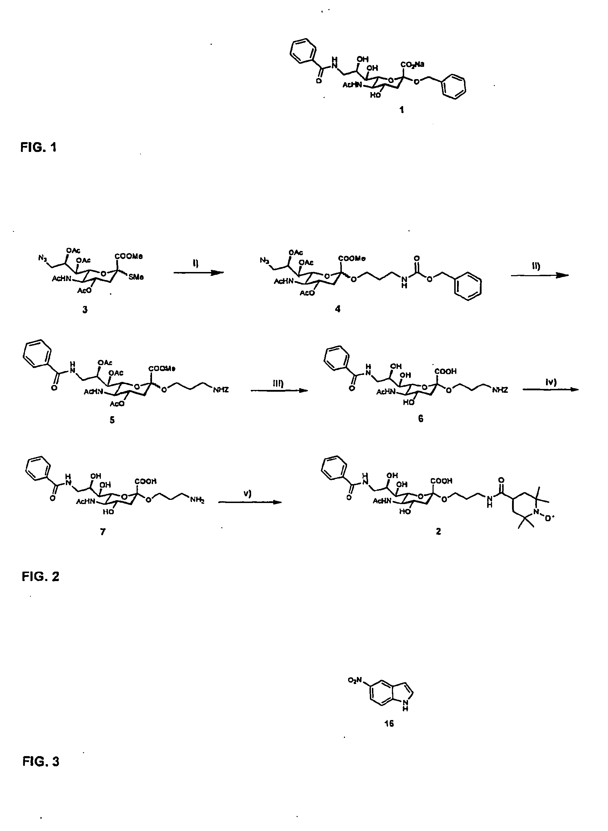

**FIG. 1** shows a micromolar MAG antagonist 1 (initial lead).
**FIG. 2** shows how second-site ligands were indentified in the presence of the spin-labeled analogue 2 of the initial lead 1: i) HO(CH₂)₃NHZ, NIS, TfOH, ACN, -40°C to -30°C, 14 h; ii) PHCOCl, PPh₃, DCE, r.t. 14 h, 95%; iii) a) NaOMe, MeOH, r.t. 2 h; b) H₂O, 2 h, 52%; iv) [H]₂, Pd/C, MeOH, 2 h, 95%; v) a) Dhbt, DIC, THF, 0°C, 2 h; b) 4-carboxy-TEMPO, DMF, r.t. 2 h, 57%.
**FIG. 3** shows a hit from a second-site library.

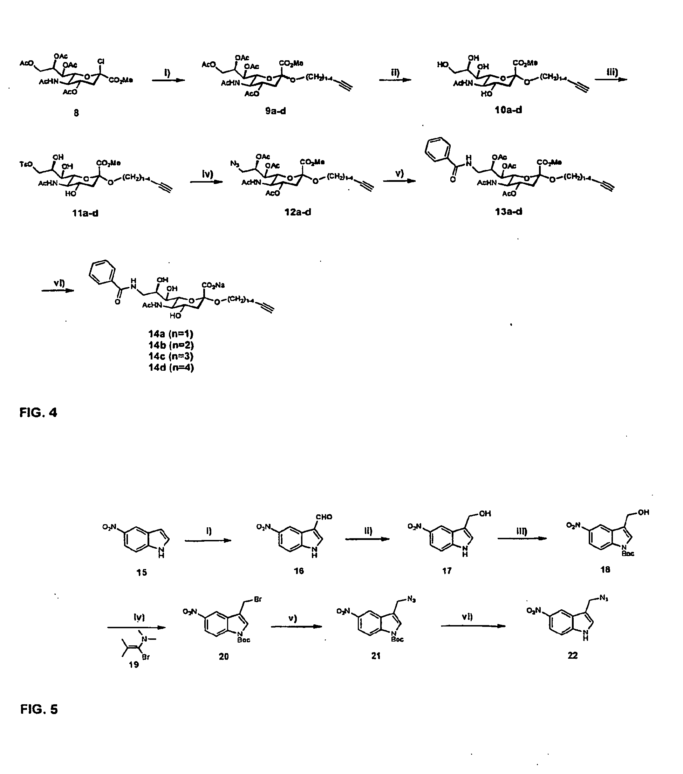

**FIG. 4** shows the reaction in which the initial lead was substituted with linkers of varying length resulting in compounds **14a** to **14d**: i) AgOTf, ACN, MS 3 A, r.t. alkinol; ii) 1M NaOMe, MeOH, Amberlyst 15 H⁺, r.t. 2 h; iii) pTsCl, pyr, 0°C; iv) a. NaN₃, DMF, 60°C, 24 h; b. Ac₂O, pyr, DMAP; v) PhCOCl, PPh₃, DCE, r.t. 16 h; vi) 10%NaOH, MeOH, Dowex 50X8 (Na⁺).

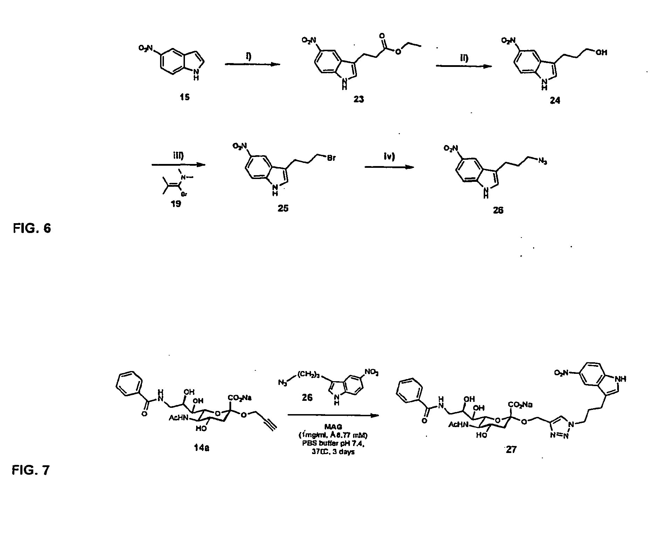

**FIGS. 5** and **6** show the reactions in which the second-site ligand was substituted with linkers of varying length resulting in compounds **22** and **26.** Reactions leading to compounds **22** (FIG. 5): i) POCl₃, DMF, 92%; ii) NaBH₄, MeOH, 93%; iii) Boc₂O, DMAP, DCM, 14 h, 62%; iv) **19,** CHCl₃, r.t. 6 h, 62%; v) NaN₃. 15-C-5, DMF, r.t. 16 h, 95%; vi) TFA, DCM, 70%. Reactions leading to compounds 26 (FIG. 6): i) a. MA, HCHO, proline, ACN, r.t. 20 h; b. Cu, pyr, EtOH, reflux, 2 h; ii) LAH, THF, 82%; iii) **19,** CHCl₃, r.t. 6 h; iv) NaN₃, 15-C-5, DMF, r.t. 16 h, 96%.
**FIG. 7** shows the reaction product (hit) resulting from incubating MAG with a mixture of the four compounds 14a to 14d ("substituted initial lead") with the two substituted second-site ligands. MAG selected 14a and 26 to produce compound 27.

### SUMMARY OF THE INVENTION

The present invention is, in one embodiment, directed at a method for producing a new lead for a drug candidate comprising providing a target and at least one second-site ligand. An initial lead binds to a first site at the target and the ligand binds to a second site at the target. A second-site ligand affected by the initial lead is identified by a spin label attached to the initial lead by identifying a second-site ligand whose NMR signal and/or paragenetic relaxation is quenched or enhanded, respectively. A homogenous pool of the initial lead, in which each initial lead is substituted with a first linker comprising a first functional group is combined with a homogenous pool of the identified second-site ligand, in which each identified second-site ligand is substituted with a second linker comprising a second functional group. The first and second linker form a covalent linkage between the initial lead and said second-site ligand to produce a new lead for a drug candidate if
(1) the initial lead and the second-site ligand are bound to the target, and
(2) the first and second functional groups are oriented so that they react with each other.

Then the lead for a drug candidate wherein the initial lead is covalently linked to said second-site ligand is identified.

The first and second linker may vary in length or type and, optionally, functional group between initial leads/indentified second-site ligands in the respective homogenous pool.

The target may have a structure that is unknown.

The initial lead is substituted with a spin label, such as a TEMPO-derivate, to quench a NMR signal of the second-site ligand, when said second-site ligand is bound to the target.

The second-site ligand may bind to the target proximate of the initial lead, such as in a distance of less than 1, 2, 3, 4, 5, 6, 7, 8, 9, 10, 11, 12, 13, 14, 15, 16, 17, 18, 19, 20, 21, 22, 23, 24, 25, 36, 27, 28, 29 or 30 A. The first and second functional group may react with each other in a chemical reaction, such as a cycloaddition reaction, to form a covalent linkage.

The first functional group may be an azide, the second functional group may be an acetylene or vice versa.

The initial lead and/or said second-site ligand may have micromolar (µM) or milimolar (mM) binding affinities to the target, while the new lead for a drug candidate formed may have a binding affinity to the target exceeding a sum of the binding affinities of the initial lead and the second-site ligand, such as a micromolar (µM) or nanomolar (nM) binding affinity. The second-site ligand may be part of a pool of compounds having a framework found in known drugs or having a set of properties found in drugs. The initial lead may be substituted with a spin label and may quench a NMR signal of the second-site ligand when said second-site ligand is bound to the target. This pool of compounds may be subdivided into sub-pools, wherein the sub-pool may be selected so that, in an NMR spectrum of the sub-pool at least one signal of each second-site ligand in said sub-pool remains distinguishable.

The initial lead may also be identified as part of the above method. This initial lead may, in such an embodiment, be selected from a pool of compounds having a framework found in known drugs or having a set of properties found in drugs.

The disclosure also describes a kit for producing new leads for drug candidates comprising: a homogenous pool of compounds having a framework found in known drugs or having a set of properties found in drugs, wherein each of said compounds in said pool has at least one linker arm attached to it; and instructions for selecting from said compounds a second-site ligand for producing a new lead for drug candidates according to the methods described herein. The compounds of the homogenous pool may comprise linker that vary in length or type and, optionally, functional group.

The invention is useful for finding new leads for drug candidates produced via the above method, including a drug candidate or drug comprising or being based on the following compound, which may be further optimized:

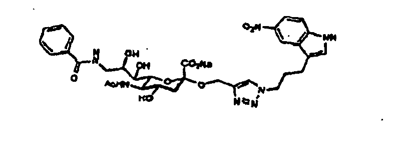

### DETAILED DESCRIPTION OF VARIOUS AND PREFERRED EMBODIMENTS OF THE INVENTION

**A ligand** such as a second-site ligand and an initial lead is, in the context of the present invention, any molecule that may bind to a target. Preferably, the ligand is a relatively small molecule ("fragment'), particularly a small organic molecule of less than about 2000 Da. Especially preferred are "drug-like" molecules. Ligands may be naturally derived ligands, e.g. obtainable by isolation from a natural organism, preferably from plants, animals or human beings or obtainable by genetic engineering. Such ligands may further be modified, e.g. by treating them with enzymes or chemical compounds.

**A new lead for drug candidates,** in the context of the present invention, is a modular molecule that may serve as a base structure for the design of a new drug or may itself constitute a new drug. A drug candidate may comprise or be based on such a new lead, usually, but not always, the new lead for drug candidates is further optimized, e.g., by chemical modification aimed at, among others, improvement of potency, selectivity vs. related targets, reduction of toxicity, metabolic susceptibility, solubility, stability and, last but not least *in vivo* efficacy.

The term **second-site ligand,** in the context of the present invention, is used broadly and means any ligand that binds the target in addition to a first ligand or first-site ligand. Such a second-site ligand may be a "true" second-site ligand, that is a ligand that binds to the target simultaneously with and in the proximity of the first ligand. A first ligand that binds to a target and has characteristics, such as certain Inhibitory or blocking effects, that make it a potential module for a drug lead candidate can be called an **initial lead.**

A **homogenous pool** of a ligand ("homogenous library" of a ligand), e.g. of a second-site ligand or an initial lead according to the present invention means that the ligands in the pool are the same but may vary in the nature of their linker, such as length, type and/or functional group, including how and where the linker is attached to the ligand.

A second-site ligand is said to be **affected** by the initial lead if the initial lead exerts, with or without prior modification, an influence on the second-site ligand which can be measured and is, in certain embodiments, quantifiable.

Second-site ligands that are affected by the initial lead can be detected in different ways, including NMR based methods that involve, e.g. spin labeling the initial lead. **Spin labels** according to the present invention include radicals such TEMPO radicals. In a preferred embodiment, the second-site ligand is detected via the method described by Jahnke et al. (Jahnke, 2000). In a preferred embodiment, the reaction solution for identifying second-site ligands comprises not more than about 200 µM, not more than about 100 µM, not more than about 75 µM, not more than about 50 µM, not more than about 25 µM, not more than about 10 µM, not more than about 9 µM, not more than about 8 µM, not more than about 7 M, not more than about 6 µM, not more than about 5 µM, not more than about 4 µM, not more than about 3 µM, not more than about 2 µM or not more than about 1 µM of target. Preferably the reaction solution co prises between about 25 µM and about 5 µM, between about 10 µM and about 1 µM and more preferably between about 10 µM and about 5 µM of target. Jahnke's method detects the paramagnetic relaxation enhancement on a second ligand caused by a spin -labeled first ligand. The method allows for the detection of "true" second-site ligands, which are ligands that bind to the target (1) simultaneously with and (2) in the proximity of the first ligand ("second-site screening").

In one preferred embodiment of the present invention, the synthesis requirements associated with the method of the present invention will be limited. For example, starting with a library that is commercially or otherwise available, the number of compounds that need synthesis, such as the attachment of a spin label, may be equal or less than 15, 14, 13, 12, 11, 10, 9, 8, 7, 6, 5, 4, or 3. If a kit according to the present disclosure is used, the number may be even less, namely 2 or even only 1.

However, as the person skilled in the art will appreciate that other methods not falling within the scope of this invention can be employed to determined whether a second-site ligand is affected by the initial lead. These methods include, but are not limited to, mass spectroscopical methods including "SAR by MS", X-ray crystallography as well as target-based assays. In the latter approach, a second-site ligand that is situated in the proximity of, e.g. an initial lead, can be identified by observing changes in its properties, such as binding properties, due to the proximity of a bound initial lead.

The second-site ligand is, in certain embodiments of the present invention, part of a pool of compounds having a framework found in known drugs and having a set of properties found in drugs ("drug-like compounds"). These pools, which are sometimes also referred to as "libraries," may be established following a wide array of parameters and/or filtering methods. For example, it has been suggested to avoid compounds that contain atoms other than C, O, H, N, S, P, F , Cl, Br, I or larger molecules. Especially predictive for good oral bioavailability is the "Lipinski rule of five" (Sen, 2006). Other criteria for library design are: diversity, drug-like character, solubility and synthetic accessibility. Information on frameworks may also be used to design a fragment library for, e.g., NMR screening by choosing actual ring/substructures that match the most frequently occurring frameworks. The SHAPES NMR fragment library describes common frameworks for molecules that are often found in drugs.

The second-site ligand is said to bind **proximate** to ( "in the proximity") the initial lead if, subsequent to binding to the target, the second-site ligand is affected by the initial lead or vice versa. As the person skilled in the art will appreciate, what is considered proximate will generally depend on the structure of the target. A second-site ligand is typically, but not exclusively, said to bind "proximate" to the initial lead when it binds at a distance of less than about 1, 2, 3, 4, 5, 6, 7, 8, 9, 10, 11, 12, 13, 14, 15, 16, 17, 18, 19, 20, 21, 22, 23, 24, 25, 36, 27, 28, 29 or 30 A, preferably at a distance of less than about 25 A, even more preferably at a distance of less than about 20 A.

As the person skilled in the art will appreciate the **initial lead** can be identified in a wide variety of ways. For example, a physiological ligand that is detected or known can serve as an initial lead. The initial lead can also be detected using classical screening techniques including an array of high-throughput screening approaches. A wide variety of NMR based techniques are also available and include, but are not limited to, nuclear Overhauser effect (NOE) (Meyer, 1997; Chen, 1998; Chen, 2000; Fejzo, 1999), chemical shift perturbation (Shuker, 1996), diffusion (Lin, 1996; Lin 1997-a; Lin, 1997-b), relaxation (Hadjuk, 1997) and saturation transfer (Mayer, 1999; Klein, 1999; Dalvit, 2000).

A **target** can be any kind of biomolecule that is amenable to influence by the binding of another molecule. Such a target may be a nucleic acid, e.g. a DNA or RNA molecule, or may be a peptide or polypeptide such as a protein (including a glyco- or lipoprotein). Typical categories of targets include, but are not limited to, enzymes, receptors, transporters and channels. In certain embodiments, the target is known to have a function in disease onset, development or establishment. In many, but not all embodiments, the target is naturally derived.

The structure of a target is said to be **unknown** if structural parameters of the target that characterize a second binding site have not sufficiently been characterized to allow a rational design of a limited pool of second-site ligands for this binding site and/or of klinkages between first and second-site ligands. This is often the case when the crystal structure of the target has not been established. The pool of compounds that is selected for testing such a unknown target may comprise about 0.5 million molecules or more, about 0.4, about 0.3, about 0.2, or about 0.1 million molecules or more. In certain embodiments, the pool may include about 50.000 or more, about 10.000 or more, about 5.000 or more, 1.000 or more fragments. These numbers assume that the pool has not been reduced in size by selecting "drug-like compounds." As the person skilled in the art will appreciate, not all of the molecules in such a pool need to be tested to find a second-site ligand. Also, the pool may be subdivided in smaller sub-pools. Even if the target is known, the method of the present invention can be performed without structural information about the target.

The ligand binding site(s), in particular the "second site" on the target may be a flat (unstructured) binding site that might be as large as 1000 Å or more. However, the area of such a binding site is typically less than about 1000 Å, less than about 900 A, less than about 800 Å, but more than about 100 Å, more than about 200 Å, more than about 300 Å, more than about 400 A or more than about 500 Å, preferably it is between about 500 A and about 1000 Å. These binding sites comprise, but are not limited to, those involving carbohydrate-lectin interactions but also those involving protein-protein interactions. In one embodiment of the present invention, the method of the present invention is employed, when optimization via approaches more suitable for structured binding sites, such as mimetic approaches, as in the context of, e.g. SLe^{x}-E-selectin, are unsuccessful.

A linker ("linker arm") according to the present invention usually comprises an arm section and a functional group. This functional group may react with another functional group to form a covalent linkage between two fragments such as an initial lead ("first ligand"/ "first-site ligand") and a second-site ligand of a target. In certain embodiments one of the linkers comprises only a functional group. However, typically, the covalent linkage is formed by two linkers each comprising an arm section and a functional group. Each of the linkers is attached to the initial lead and the second-site ligand, respectively. In one preferred embodiment, the functional groups, located at the distal ends of the arm section of the linker, are able to react with each other under a certain set of conditions. For example, the functional groups can react with each other when the distance between them is small enough for them to react. This distance will also be referred to as "**reaction distance**". The ability to react might be influenced by a variety of factors such a temperature, presence and absence of co-factors and similar. These factors, as the person skilled in the art will appreciate, depend on the nature of the functional groups involved. As the person skilled in the art will also appreciate, the type of linker might vary widely. Two or more linker are said to vary in type, if, as will be explained in the next section, the arm section of one linker differs in composition from the arm section of the other linker. For example, one type of linker comprises a simple hydrocarbon chain as an arm section, while the other type comprises a sulfur containing aliphatic arm section and yet another type comprises a heteroaromatic arm section. Varying the length of the linker is often accomplished by varying the length of the arm section of the linker. Thus, a linker having an arm section of the formula (CH₂)₃ will vary in length from a linker having an arm section of the formula (CH₂)₄.

The arm section of a linker can comprise a wide variety of atoms. In one preferred embodiment, the arm section will comprise a simple hydrocarbon chain and can be described by the structural formula (CH₂)ₙ wherein n can be any number, but is preferably 2, 3, 4, 5, 6, 7, 8, 9, 10, 11, 12, more preferably between 2 and 6. However, other arm section designs are also within the scope of the present invention. For example, the arm section might contain heteroatoms such as oxygen, sulfur or nitrogen. In a preferred embodiment, these heteroatoms alternate with at least two consecutive C atoms to form a chain of atoms. The arm section can also comprise ring structures such as aliphatic, heteroaliphatic, aromatic or heteroaromatic rings. While these are some examples, the person skilled in the art will appreciate that arm section design can vary widely. However, arm sections that result in few or no conformational restrictions will, in most embodiments, be preferred over arm sections that cause a variety of conformational restrictions. In certain embodiments, the arm sections are attached to an atom at the target that is accessible to such attachment. In certain preferred embodiments such an atom is proximal to the other ligand. Depending on the attachment point, the reactions involved in such an attachment, which often result in covalent bonds, will vary. However, a stable attachment is in many embodiments of the invention preferred over a non-stable attachment.

The **functional groups** at the initial lead and the second-site ligand are able to react with each other. In a preferred embodiment, these functional groups engage in "click chemistry" reactions, which include, but are not limited to, cycloaddition reactions, such as hetero-Diels-Adler and 1,3 dipolar cycloaddition reactions; reactions of carbonyls, e.g. the formation of hydrazones, oxime ethers and heteroaromatic synthesis; addition to carbon-carbon multiple bonds or nucleophilic substitution on strained compounds and intermediates (Röper & Kolb, 2006). In a particularly preferred embodiment one of the functional groups is an azide group and the other is an acetylene group.

Whether the first and second functional group attached to the initial lead and the second-site ligand respectively, **are oriented so that they react with each other** will, in many embodiments of the present invention, depend on the length of the arm section of the linker of the initial lead and/or second-site ligand. In certain embodiments it will also depend, alternatively or additionally, on the composition of the arm section.

The invention will now be described by a non-limiting example. In particular, the search of a new lead for a drug candidate acting on myelin-associated glycoprotein (MAG) (McKerracher, 1994; Mukhopadhyay, 1994; Vinson, 2001) will be described..The person skilled in the art will readily be able to apply the principles used in the approach described herein for MAG to other biomolecules, in particular molecules, such as proteins, whose characterization is somewhere at the level described below for MAG.

MAG is a sialic acid-binding immunoglobulin-like lectin and a member of the Siglec family. Its role (Kelm, 1994; Crocker, 1998) as one of several myelin components inhibiting axonal regrowth after injury has drawn a lot of attention (McKerracher, 2002). Although the exact mechanism is still unclear, it is believed that blocking the inhibitory activity of MAG could support the regeneration after injury to the central nervous system (CNS). Schnaar (Yang, 1996) reported that GQ1bα, GD1a and GT1b, known to be expressed on myelinated neurons in vivo, are functional ligands of MAG. These gangliosides have been synthesized in preparative amounts (Hotta, 1995; Ito, 1999; Ito, 2001), and were therefore used to establish a structure affinity relationship (SAR). Thereafter, the SAR profile was refined by numerous synthetic contributions based on ganglioside fragments (Kelm, 1998; Strenge, 1998; Sawada, 1999; Schwizer, 2006; International Patent Publication WO03/000709; U.S. Patent Publication 2004176309), mimics thereof (Gao, 2007) and neuraminic acid derivatives (Schwizer, 2006). The recently reported ability to reverse MAG inhibition with monovalent glycosides (Vyas, 2005) encourages further exploration of glycans and glycan mimics as inhibitors of MAG-mediated axonal outgrowth inhibition.

A first generation of MAG blockers with micromolar affinity was obtained when ganglioside GQ1bα could successfully be reduced to a low molecular weight ligand consisting only of a modified N-acetylneuraminic acid moiety (Kelm, 1998; Strenge, 1998; Sawada, 1999; Schwizer, 2006; Collins, 1999; International Patent Publication WO03/000709; U.S. Patent Publication 2004176309). However, a broad optimization effort did not result in nanomolar MAG antagonist (Gao, 2007; Schwizer, 2006). Since the crystal structure of MAG is not yet available, no rational design approach was possible. In addition, a homology model (Bhunia, 2007) based on the crystal structure of the N-terminal V-set domain of sialoadhesin (May 1998), revealed a flat, unstructured binding site and no obvious possibility for additional intruding interactions with the protein surface. Inspection of the homology models did not allow the identification of proximate second sites on the MAG surface.

Therefore, a two-stage combinatorial approach for the design of low molecular weight antagonists was explored. The starting point was the micromolar MAG antagonist 1 shown in **FIG. 1****,** a sialic acid derivative, which has been shown to exhibit affinity in the low micromolar range (Kelm, 1998; International Patent Publication WO03/000709; U.S. Patent Publication 2004176309; Shelke, 2007). **1 was** selected as ligand for the first binding site ("initial lead"). In the first step, second-sites located in the proximity of the sialic acid binding site were identified applying the elegant approach described by Jahnke *et al.* (Jahnke, 2000). Members of a library composed of 60 divers or sub-libaries thereof, which are drug-like, soluble and synthetically easily available fragments ("second-site ligands") binding to the target protein were identified based on their shorter relaxation time in H-NMR. Then, in the presence of the spin-labeled analogue **2** of the first ligand **1** that is shown in the scheme depicted in **FIG. 2** neighboring second-site ligands were identified according to their paramagnetic relaxation enhancement caused by the spin-labeled first-site ligand **2.** Since, in a target-based inhibition assay (Crocker, 1996), **1** and **2** exhibited comparable affinities (approx 20 µmolar), it was unlikely that the modification with the spin label would induce a drastic change in the binding mode. For the fragment **15** shown in **FIG. 3****,** enhanced relaxation was observed indicating binding proximate to (radius of 15 to 20 A) the unpaired electron of the Tempo spin label in **2.** The affinity of this fragment **15** in a surface plasmon resonance experiment was determined to be in the low millimolar range.

In the second step, the first and second-site ligands were linked using the *in situ* click chemistry approach (Lewis, 2002; Bourne, 2004; Manetsch, 2004; Mocharla, 2005), which allows the irreversible target-guided synthesis of high affinity ligands from small fragments. For that purpose, a small homogenous library of the first-site ligand, the sialic acid derivatives **14a-d** shown in scheme depicted in **FIG. 4****,** was synthesized. To avoid large entropic costs originating from numerous rotatable bonds (Mammen, 1998) present in extended linkers, only short chains allowing the connection of spatially close ("proximate") binding sites were considered.

As a second-site ligand, 5-nitro-1H-indole **(15),** was selected as the nitro group which allows for further exploration of the MAG surface. Therefore, the two azido compounds, 3-azidomethyl-5-nitro-1H-indole 22 shown in the scheme depicted in **FIG. 5** and 3-azidopropyl-5-nitro-1H-indole **26** shown in the scheme depicted in **FIG. 6****,** were synthesized by standard procedures.

For the *in situ* click chemistry experiment (Lewis, 2002; Bourne, 2004; Manetsch, 2004; Mocharla, 2005), Fc-MAG(d1-3) (Crocker, 1996) (8.77 µM) was incubated with a mixture of the four substituted first-site ligands **14a-d** (each 40 µM) and the two substituted second-site ligands **22, 26** (each 800 µM) in phosphate buffer (pH 7.4) at 37 °C for 24 h. The different concentrations of the first- and second-site ligands reflect their affinities in the low micro- and millimolar range. LC-MS analysis indicated according to the MW that only one new compound, namely the trizole **27** shown in **FIG. 7** was formed. **27** was independently synthesized and its affinity determined in a SPR experiment where Fc-MAG(d1-3) (Crocker, 1996) was immobilized by protein A interaction giving a K_{D} of 190 nM. To exclude that false positives are formed during incubation, two control experiments were performed. In the first, MAG was replaced by bovine serum albumin. In the second, no protein was added. In both experiments, no triazole formation could be observed.

In sum, a nanomolar MAG antagonist could be surprisingly identified with the method of the present invention. The synthetic effort was, compared to other methods, minimal. Starting with the library composed of 60 divers, merely 6 compounds needed to be synthesized (including substituted first and second-site ligands) to successfully identify a high affinity antagonist for MAG. No structural information of the target protein was required.

### MATERIALS AND METHODS

### Synthesis of the sialic acid derivatives 14a-d

**Methyl (5-acetamido-4,7,8,9-tetra-*O*-acetyl-2-chloro-3,5-dideoxy-D-*glycero*-D-*galacto*-2-**nonulopyranosid)onate (8). To a stirred solution (-20°C) of NeuNAc (800 mg, 1.50 mmol) and AcCl (1.8 ml) in DCM (5 ml) was added 32% HCl (213 ml). After stirring for 16 h at r.t., the mixture was diluted with cold DCM and subsequently washed with H₂O, 5% NaHCO₃ and brine. The organic layer was dried over Na₂SO₄ and evaporated to give **8** (700 mg, 91 %) as a fluffy solid.

¹H NMR (500 MHz, CDCl₃): δ 1.95 (s, 3H, NHAc), 2.08, 2.09, 2.11, 2.16 (4s, 12H, OAc), 2.32 (t, *J* = 13.9 Hz, 1H, H-3a), 2.82 (dd, *J* = 4.8, 13.9 Hz, 1H, H-3e), 3.91 (s, 3H, COOCH₃), 4.10 (dd, *J* = 5.7, 12.5 Hz, 1H, H-9a), 4.24 (q, J = 10.5, 1H, H-5), 4.38 (dd, J = 2.4, 10.8 Hz, 1H, H-6), 4.45 (dd, J = 2.7, 12.6 Hz, 1H, H-9b), 5.21 (m, 1H, H-8), 5.36 (d, J = 10.2 Hz, 1H, NH-5), 5.45 (m, 1H, H-4), 5.51 (dd, J = 2.4, 7.1 Hz, 1 H, H-7). (See, e.g., Claridge, **1999**).

**Methyl [(2-propinyl) 5-acetamido-4,7,8,9-tetra-*O*-acetyl-3,5-dideoxy-D-*glycero*-α- and β-D-*galacto*-2-nonulopyranosid]onate (9a).** Compound 8 (1.40 g, 2.75 mmol) was dissolved in dry MeCN (30 ml) containing propargyl alcohol (308 mg, 5.50 mmol) and molecular sieves 3A (4.0 g). The mixture was stirred at r.t. for 1 h with light exclusion. Then AgOTf (1.41 g, 5.50 mmol) was added in one portion and stirring continued at r.t. for 16 h. The mixture was filtered through a pad of Celite and the Celite was washed thoroughly with DCM. The filtrate was subsequently washed with 10% NaHCO₃, sat. Na₂S₂O₃ and water. The organic layer was dried with Na₂SO₄ and the DCM evaporated. The residue was purified by silica gel column chromatography (PE:DCM:iPrOH 8:4:1). 25a (1.150 g, 79%) was obtained as anomeric mixture (α:β 1:1).

**Methyl [(2-propinyl) 5-acetamido-3,5-dideoxy-D-*glycero*-α-D-*galacto*-2-nonulopyranosid]onate (10a).** A solution of **9a** (1.15 g, 2.20 mmol) in dry MeOH (30 mL) was treated with 1 M NaOMe/MeOH (5 mL) at r.t. for 2 h. The reaction mixture was neutralized with Amberlyst 15 (H⁺) ion-exchange resin and filtered through a pad of Celite. The Celite was washed thoroughly with methanol (3 x 10 mL), and the combined filtrates were evaporated to dryness and purified by silica gel column chromatography (DCM/MeOH 7:3). The initial fractions collected gave **10a** (263 mg, 62%). ¹H NMR (500 MHz, CD₃OD): δ 1.71 (t, *J* = 12.6 Hz, 1H, H-3a), 1.97 (s, 3H, NHAc), 2.67 (dd, *J* = 4.6, 12.8 Hz, 1 H, H-3e), 2.83 (X cf ABX, *J* = 2.4 Hz, 1 H, C≡CH), 3.47 (dd, *J* = 1.3, 9.0 Hz, 1 H, H-7,), 3.56 (dd, *J* = 1.3, 10.5 Hz, 1H, H-6), 3.60-3.65 (m, 2H, H-4, H-9a), 3.73-3.83 (m, 3H, H-5, H-8, H-9b), 3.81 (s, 3H, CO₂CH₃), 4.32 (AB of ABX, J= 2.4, 15.9 Hz, 2H, H-1'); ¹³C NMR (125 MHz, D₂O): δ 23.2 (NHCOCH₃), 41.9 (C-3), 53.3 (C-1'), 53.9 (CO₂CH₃), 54.1 (C-5), 65.1 (C-9), 68.9 (C-4), 70.5 (C-7), 72.6 (C-8), 75.3 (C≡CH), 75.3 (C-6), 80.7 (C≡CH), 99.9 (C-2), 170.9, 175.6 (2CO).

**Methyl [(2-propinyl) 5-acetamido-3,5-dideoxy-9-*O*-(4-toluenesulfonyl)-D-*glycero*-α-D-*galacto*-2-nonulopyranosid]onate (11a).** To a solution of **10a** (263 mg, 0.728 mmol) in pyridine (15 ml) was added p-TsCl (138 mg, 0.728 mmol) at 0°C. After 2 h, additional *p*-TsCl (69 mg, 0.364 mmol) was added and stirring continued for 16 h at 5°C. The reaction mixture was warmed to r.t. and then diluted with methanol (20 mL) and stirring continued for 30 min. After removal of the solvents the remaining syrup was purified by chromatography on silica gel (DCM/MeOH 19:1) to yield **11a** as a foamy solid (223 mg, 60%).

¹H NMR (500 MHz, CD₃OD): δ 1.69 (t, *J* = 12.8 Hz, 1H, H-3a), 1.99 (s, 3H, NHAc), 2.46 (s, 3H, Ph-CH₃), 2.67 (dd, *J* = 4.6, 12.8 Hz, 1H, H-3e), 2.84 (X of ABX, *J* = 2.5 Hz, 1H, C≡CH) 3.44 (dd, *J* = 1.4, 8.8 Hz, 1H, H-7), 3.55 (dd, *J* = 1.4, 10.5 Hz, 1H, H-6), 3.63 (m, 1H, H-4), 3.71 (m, 1H, H-5), 3.81 (s, 3H CO₂CH₃), 3.97 (m 1H, H-8), 4.08 (m, 1H, H-9a), 4.23-4.32 (m, 3H, H-9b, H-1'), 7.45 (AA' of AA'BB', 2H, *J* = 8.0 Hz, Ar-H), 7.80 (BB' of AA'BB', 2H, *J* = 8.3 Hz, Ar-H); ¹³C NMR (125 MHz, CD₃OD): δ 22.0 (Ph-CH, 23.1 (NHCOCH₃), 41.9 (C-3), 53.4 (C-1'), 53.7 (CO₂CH₃), 54.0 (C-5), 68.7 (C-4), 70.1 (C-8), 70.4 (C-7), 74.1 (C-9), 75.0 (C-6), 76.1 (C≡CH), 80.8 (C≡CH), 100.0 (C-2), 129.5, 131.4, 134.7, 146.8 (6C, C₆H₅), 170.5, 175.5 (2CO).

**Methyl [(2-propinyl) 5-acetamido4,7,8-tri-*O*-acetyl-9-azido-3,5,9-rideoxy-D-*glycero*-α-D-*galacto*-2-nonulopyranosid]onate (12a).** A mixture of 11a (223 mg, 0.18 mmol), crown ether 18-C-6 (44.8 mg, 0.17 mmol) and NaN₃ (139.7 mg, 2.15 mmol) was stirred in DMF (4 mL) at 60°C for 24 h. The mixture was filtered through a pad of Celite and the filtrate was evaporated to dryness. Purification by column chromatography on silica gel in DCM:MeOH (7:3) yielded a white solid (110 mg, 66%), which was dissolved in dry pyridine (1.17 ml, 14.8 mmol) and stirred at 0°C for 15 min under argon before DMAP (4.90 mg, 0.04 mmol) and Ac₂O (1.28 ml, 12.6 mmol) were added simultaneously at 0°C. After stirring at r.t. for 14 h, TLC analysis showed completion of the reaction. After evaporation of the solvents and chromatography on silica gel (DCM/MeOH 9:1) **12a** was obtained as a white foam (90 mg, 62%).

¹H NMR (500 MHz, CDCl₃): δ 1.84 (s, 3H, NHAc), 1.95 (t, *J* = 12.5 Hz, 1H, H-3a), 2.00, 2.12, 2.14 (3s, 9H, 30Ac), 2.43 (X of ABX, *J* = 2.4 Hz, 1H, C≡CH), 2.60 (dd, *J* = 4.7, 12.9 Hz, 1 H, H-3e), 3.25 (dd, *J* = 2.9, 5.2 Hz, 1H, H-9a), 3.55 (dd, *J* = 2.5, 14.7 Hz, 1H, H-9b), 3.78 (s, 3H, CO₂CH₃), 4.06 (m, 2H, H-5, H-6), 4.12, 4.35 (AB of ABX, *J* = 2.4, 15.7 Hz, 2H, H-1'), 4.83 (m, 1H, H-4), 5.28 (m, 2H, H-7, H-8), 5.48 (d, *J* = 9.3 Hz, 1H, NH); ¹³C NMR (125 MHz, CDCl₃): δ 21.2, 21.2, 21.4 (30COCH₃), 23.5 (NHCOCH₃), 38.1 (C-3), 49.5 (C-5), 51.2 (C-9), 53.1 (CO₂CH₃), 53.3 (C-1'), 68.2 (C-8), 69.1 (C-4), 70.2 (C-7), 73.3 (C-6), 75.0 (C≡CH), 79.2 (C≡CH), 98.5 (C-2), 168.1, 170.6, 170.6 170.7, 171.3 (5CO).

**Methyl [(2-propinyl) 5-acetamido-4,7,8-tri-*O*-acetyl-9-benzamido-3,5,9-trideoxy-D-glycero-α-D-galacto-2-nonulopyranosid]onate (13a).** Compound **12a** (90 mg, 0.17 mmol) was reacted with benzoyl chloride (95.2 mg, 0.68 mmol) and triphenyl phosphine (96.9 mg, 0.37 mmol) in DCE (4 mL) for 1 h. After stirring at r.t. for 16 h, the solvent was evaporated. Purification by column chromatography on silica gel (DCM:MeOH 97:3) yielded **13a** as a solid (54 mg, 52 %).

¹H NMR (500 MHz, CDCl₃): δ 1.88 (s, 3H, NHAc), 1.98 (t, *J* = 12.6 Hz, 1H, H-3a), 2.03, 2.09, 2.24 (3s, 9H, 30Ac), 2.43 (X of ABX, *J* = 2.4 Hz, 1H, C≡CH), 2.64 (dd, *J* = 4.6, 12.7 Hz, 1H, H-3e), 2.97 (m, 1H, H-9a), 3.73 (s, 3H, CO₂CH₃), 3.99 (dd, *J* = 2.1, 10.8 Hz, 1H, H-6), 4.16 (m, 2H, H-5, H-1'a), 4.32 (m, 1H, H-9b), 4.39 (dd, *J* = 2.5, 15.6 Hz, 1H, H-1'a). 4.85 (m, 1H, H-4), 5.16 (dd, *J* = 2.0, 9.8 Hz, 1H, H-7), 5.27 (m, 1H, H-8), 5.60 (d, *J* = 10.1 Hz, 1H, NH-5), 7.11 (m, 1H, NH-9), 7.44, 7.58, 7.81 (m, 5H, C₆H₅); ¹³C NMR (125 MHz, CDCl₃): δ 21.2, 21.4, 21.5 (30COCH₃), 23.4 (NHCOCH₃), 38.1 (C-3), 38.8 (C-9), 49.7 (C-5), 53.2 (2C, CO₂CH₃, C-1'), 68.2 (C-7), 68.7 (C-8), 69.3 (C-4), 72.6 (C-6), 74.9 (C≡CH), 79.2 (C≡CH), 98.4 (C-2), 127.3, 128.8, 130.4, 133.8 (6C, C₆H₅), 167.9, 167.9, 170.8 171.1, 171.6, 172.7 (6CO).

**Sodium [(2-propinyl) 5-acetamido-9-benzamido-3,5,9-trideoxy-D-glycero-α-D-galacto-2-nonulopyranosid]onate** (14a). To a solution of compound **13a** (54 mg) in methanol (2 mL) was added 10% aq. NaOH (0.2 mL). The mixture was stirred at r.t. for 3 h. After neutralization with 10 % HCl (0.2 ml), the solution was concentrated and the residue was purified by reversed-phase chromatography (RP-18 column, 5% gradient MeOH in water), Dowex 50X8 ion-exchange chromatography (Na⁺ type), and P2 size exclusion chromatography to afford **14a** (41 mg, 95%) as a colorless solid after final lyophilization from water.

[α]_{D}-0.5 (c 0.10, H₂O); ¹H NMR (500 MHz, D₂O): δ 1.67 (t, *J* = 12.2 Hz, 1H, H-3a), 1.98 (s, 3H, NHAc), 2.73 (dd, *J* = 4.7, 12.5 Hz, 1H, H-3e), 3.54 (m, 2H, H 7, H-9a), 3.67 (m, 1H, H-4), 3.75 (m, 2H, H-6, H-9b), 3.83 (m, 1H, H-5), 4.06 (m 1H, H-8), 4.35, 4.21 (A, B, of AB, *J* = 15.6 Hz, 2H, H-1'). 7.50, 7.58, 7.74 (m, 5H, C₆H₅); ¹³C NMR (125 MHz, D₂O): δ 22.3 (NHCOCH₃), 40.6 (C-3), 43.0 (C-9), 52.1 (C-5), 52.9 (C-1'), 68.4 (C-4), 70.1 (C-7), 70.4 (2C, C-8, C≡CH), 73.1 (C-6), 79.8 (C≡CH). 101.0 (C-2), 127.4, 129.1, 132.4, 133.9 (C₆H₅), 171.6, 173.1, 175.3 (3CO).

The sialic acid derivatives **14b, 14c** and **14d** were synthesized by the same sequence of reaction.

### 5-Nitro-1H-indole derivatives were synthesized by established procedures:

**3-(1-Azidomethyl)-5-nitro-1H-indole (22).** ¹H NMR (500 MHz, DMSO-d6): δ 4.50 (s, 2H, CH₂), 7.39 (d, *J* = 8.9 Hz, 1 H, Ar-H), 7.55 (d, *J* = 2.4 Hz, 1 H, 1H, Ar-H), 7.84 (dd, *J* = 2.3, 9.0 Hz, 1H, Ar-H), 8.45 (d, *J* = 2.3 Hz, 1H, Ar-H), 11.69 (bs, 1H, NH); ¹³C NMR (125 MHz, DMSO-d6): δ 45.9 (CH₂), 112.4, 113.2, 116.6, 117.9, 126.7, 130.3, 140.3, 141.7 (Ar-C).

**3-(3-Azidopropyl)-5-nitro-1H-indole (26).** ¹H NMR (500 MHz, CDCl₃): δ 2.02 (m, 2H, H-2'), 2.90 (t, *J* = 7.6 Hz, 2H, H-1'), 3.36 (t, J = 6.7 Hz, 2H, H-3'), 7.18 (s, 1H, Ar-C), 7.40(d, *J* = 9.0 Hz, 1H, Ar-C), 8.11 (dd, *J* = 2.1, 9.0 Hz, 1H, Ar-C), 8.48 (s, 1H, NH), 8.57 (d, J = 1.8 Hz, 1H, Ar-C). ¹³C NMR (125 MHz, MeOD): δ 22.2 (C-1'), 29.5 (C-2'), 51.1 (C-3'), 111.5, 116.6, 118.1, 118.2, 124.9, 127.2, 139.7, 141.9 (Ar-C).

### General procedure for in-situ click chemistry experiments

The protein included the Fc portion of human immunoglobulin IgG1 and the three N-terminal domains of murine MAG (for its preparation, see Crocker & Kelm (1996)). The protein used for in-situ click chemistry experiments and K_{D} determination was in PBS buffer at a concentration of 1 mg/ml and pH 7.4. The K_{D} analyses were performed at 25°C on a BIACORE 3000 surface plasmon resonance-based optical biosensor (Biacore AB, Uppsala, Sweden). Protein A (Sigma, Basel) was immobilized onto a CM5 sensor chip (Biacore AB, research grade) using standard amine coupling. All data processing and equilibrium binding constant determinations were accomplished with Scrubber^{©} (BioLogic Software, Campbell, Australia, Version 1.1 g). Kinetic data were simultaneously fit using the non-linear regression program Clamp^{©}. The equilibrium binding constants K_{D} were calculated using a 1:1 binding model. Double referencing was applied to correct for bulk effects and other systematic artifacts.

The stock solutions of alkynes **14a-14d** (10 mM in H₂O) and azides (40 mM in DMSO) were prepared. The alkynes (each 4 µl) were added to the stock solution of MAG (94 µl of 1 mg/ml solution, in PBS buffer) in an Eppendorff tube. After 2 min the azide compounds **22** and **26** (each 2 µl) were added. Then the Eppendorff tube was shaken at 37°C for 24 h.

To exclude that the triazoles obtained were false positives, two control experiments were also performed in parallel, one with BSA (94 µl of 1 mg/ml in PBS buffer) and the other in absence of a protein. In the case of 'absence of protein' only PBS buffer with pH 7.4 was used.

Samples from the above experiment were analyzed by LC-MS (reversed-phase chromatography with ESI mode spectroscopic detection in the negative ion mode with selected ion monitoring of only the expected m/z). Samples of the reactions were directly injected (10 µl). The products were identified by their retention times and molecular weights. The only newly formed product was **27.**

**Independent synthesis of 1-(5-nitro-1H-indol-3-yl-propyl)-4-{sodium [5-acetamido-9-benzamido-3,5,9-trideoxy-D-*glycero*-α-D-*galacto*-2-nonulopyranosynate-2-*O*-yl]}-methyl-1,2,3]-triazole (27).** To solution of **14a** (10 mg, 21.0 µmol) and **26** (5.14 mg, 21.0 µmol) in 'BuOH/H₂O (1:1) (0.5 ml) was added Na-ascorbate (1.2 mg, 6.00 µmol, 6 µl of a freshly prepared 1 M aqueous solution), followed by CuSO₄·5H₂O (0.15 mg, 0.60 µmol) in H₂O. This heterogeneous mixture was stirred for 36 h. Then the reaction mixture was evaporated to dryness and the residue purified by reversed-phase chromatography (LiCroPrep® RP-8, 5% gradient MeOH in H₂O). After P2 size-exclusion chromatography and lyophilization from H₂O, **27** (9 mg, 60%) was obtained as white solid.

[α]_{D} -1.4 (c 0.1, H₂O); ¹H NMR (500 MHz, D₂O): δ 1.66 (t, *J* = 12.1 Hz, 1H, H-3a), 1.87 (m, 1H, H-2"), 2.02 (s, 3H, NHAc), 2.16 (m, 2H, H-3"), 2.73 (dd, *J* = 4.6, 12.3 Hz, 1H, H-3e), 3.33 (dd, *J* = 4.0, 8.8 Hz, 1H, H-9a), 3.54 (dd, *J* = 1.4, 8.8 Hz, 1H, H-7), 3.69 (dddd, *J* = 1.9, 4.6, 7.1, 9.8 Hz, 1H, H-4), 3.77-3.87 (m, 3H, H-5, H-6, H-9b), 3.91 (dt, *J* = 2.7, 8.8 Hz, 1H, H-8), 4.14 (t, *J* = 6.3 Hz, 1H, H-1"), 4.38, 4.65 (A, B of AB, *J* = 12.0 Hz, 2H, H-1'b), 6.80 (s, 1H, Ar-H), 6.98 (d, *J* = 8.9 Hz, 1H, Ar-H), 7.16 (t, *J* = 7.6 Hz, 2H, Ar-H), 7.26 (t, *J* = 7.4 Hz, 1H, Ar-H), 7.45-7.51 (m, 4H, Ar-H), 7.54 (s, 1H, Ar-H); ¹³C NMR (125 MHz, D₂O): δ 21.1 (C-3") 22.4 (NHCOCH₃), 29.4 (C-2"), 40.6 (C-3), 43.3 (C-9), 50.1 (C-1 "), 52.1 (C-5), 57.4 (C-1'), 68.6 (C-4), 70.6 (C-7), 70.9 (C-8), 73.0 (C-6), 101.0 (C-2), 111.2, 115.7, 116.2, 116.9, 125.2, 125.7, 125.9, 127.1, 128.7, 131.7, 132.9, 139.5, 139.7 (Ar-C), 170.4, 173.4, 175.3 (3CO).

It will be appreciated that the methods of the instant invention can be in the form of a variety of embodiments, only a few of which are disclosed herein. It will be apparent to the artisan that other embodiments exist.

### Bibliography

Bhunia, A.; Gäthje, H.; Schwardt, O.; Gao, G.-P.; Kelm, S.; Benie, A.J.; Hricovini, M.; Peters, T.; Ernst, B. in preparation.
Bourne, Y.; Kolb, H.C.; Radic, Z.; Sharpless, K.B.; Taylor, P.; Marchot, P. Proc. Nat. Acad. Sci. USA 2004, 101, 1449.
Chen, A. S., M J J Am Chem Soc 1998, 120, 10258-10259.
Chen, A. S., M. J. J Am Chem Soc 2000, 122, 414-415.
Collins, B.E.; Ito, H.; Sawada, N.; Ishida, H.; Kiso, M.; Schnarr, R.L. J. Biol. Chem. 1999, 274, 37637.
Congreve, M.S.; Davis, D.J.: Devine, L.; Granata, C.; O'Reilly, M.; Wyatt, P.G.; Jhoti, H. Angew. Chem. Int. Ed. 2003, 42, 4479-4482
Crocker, P.R. and Kelm, S. (1996): Methods for studying the cellular binding properties of lectin-like receptors. In Weir's Handbook of Experimental Immunology, 5. edition, vol. 4, L. A. Herzenberg, D. M. Weir, L.A. (eds.). Herzenberg, C. (Hrsg.), Blackwell Science, Boston, 166.1.
Crocker, P.R.; Clark, E.A.; Filbin, M.T.; Gordon, S.; Jones, Y.; Kehrl, J.H.; Kelm, S.; Le Douarin, N.M.; Powell, L.; Roder, J.; Schnaar, R.L.; Sgroi, D.; Stamenkovic, I.; Schauer, R.; Schachner, M.; Tedder, T.; van den Berg, T.K.; van der Merwe, P.A.; Watt, S.M.; Varki, A. (1998) Glycobiology 8**,** Glycoforum 2 v-vi.
Dalvit, C., Fogliatto, G. P, Stewart, A, Veronesi, M, Stockman, B. J Biomol NMR 2000, 18, 65-68.
Fejzo, J.; Lepre, C. A.; Peng, J. W.; Bemis, G. W.; Ajay; Murcko, M. A.; Moore, J. M. Chem Biol 1999, 6, 755-769.
Gao, G.-P.; Smiesko, M.; Schwardt, O.; Gäthje H.; Kelm, S.; Vedani, A.; Beat Ernst, B. Bioorg. Med. Chem. 2007, 15, 7459-7469.
Hajduk, P. J., Olejniczak, E. T, Fesik, S. W. J Am Chem Soc 1997, 119, 12257-12261.
Hotta, K; Ishida, H.; Hasegawa, A. J. Carbohydr. Chem. 1995, 14, 491.
Ito, H.; Ishida, H.; Ando, S.; Kiso, M. Glycoconjugate J. 1999, 16, 585.
Ito, H.; Ishida H.; Kiso, M. J. Carbohydr. Chem. 2001, 20, 207.
Jahnke, W.; Perez, L.B.; Paris, C.G.; Strauss, A.; Fendrich, G.; Nalin, C.N. J. Am. Chem. Soc. 2000, 122, 7394.
Jahnke, W.; Flörsheimer, A.; Blommers, M.J.J.; Paris, C.G.;, Heim, J.; Nalin, C.M.;, Perez, L.B. Current Topics in Medicinal Chemistry 2003 3, 69-80.
Jencks, W.P. Proc. Natl. Acad. Sci. 1981, 78, 4046.
Kelm, S.; Peiz, A.; Schauer, R.; Filbin, M.T.; Tang, S.; de Bellard, M.E.; Schnaar, R.L.; Mahoney, J.A.; Hartnell, A.; Bradfield, P.; Crocker, P.R. Curr. Biol. 1994, 4, 965.
Kelm. S.; Brossmer, R.; Isecke, R.; Gross, H.-J.; Strenge, K.; Schauer, R. Eur. J. Biochem. 1998, 258, 663.
Klein, J., Meinecke, R, Mayer, M, Meyer, B. J Am Chem Soc 1999, 121, 5336-5337.
Lewis, W.G.; Green, L.G.; Grynszpan, F.; Radic, Z.; Carlier, P.R.; Taylor, P.; Finn, M.G.; Sharpless, K.B. Angew. Chem. Int. Ed. 2002, 41, 1053.
Lin, M., Shapiro, M. J. J Org Chem 1996, 62, 7617-7619.
Lin, M. F., Shapiro, M. J, Wareing, J. R. J Am Chem Soc 1997, 119, 5249-5250.
Lin, M., Shaprio, M. J., Wareing, J. R. J Org Chem 1997, 62, 8930-8931.
Mammen, F.; Shakhnovich, E.I.; Whitesides, G.M. J. Org. Chem. 1998, 63, 3168.
Manetsch, R.; Krasinski, A.; Radic, Z.; Raushel, J.; Taylor, P.; Sharpless, K.B.; Kolb, H.C. J. Am. Chem. Soc. 2004, 126, 12809.
A.P. May, R.C. Robinson, M. Vinson, P.R. Crocker, E.Y. Jones, Mol. Cell 1998, 1, 719-728; Brookhaven protein data bank acquisition code 1QFO.
Mayer, M., Meyer, B. Angew Chem Int Ed Engl 1999, 38.
McKerracher, L.; David, S.; Jackson, D.L.; Kottis, V.; Dunn, R.J.; Braun, P.E. Neuron 1994, 13, 805.
McKerracher, L. Proc. Natl. Acad. Sci. USA 2002, 99, 7811.
Meyer, B., Weimar, T, Peters. T. European Journal of Biochemistry 1997, 246, 705-709.
Mocharia, V.P.; Colasson, B.; Lee, L.V.; Röper, S.; Sharpless, B.K.; Wong C.-H.; Kolb, H.C. Angew. Chem. Int. Ed. 2005, 44, 116.
Mukhopadhyay, G.; Doherty, P.; Walsh, F.S.; Crocker, P.R.; Filbin, M.T. Neuron, 1994, 13, 757.
Rees D.C.; Congreve, M.; Murray, Ch.W.; Carr, R. Nature Reviews Drug Discovery 2004, 3, 660-672.
Röper, S.; Kolb, H.C.; Fragment-based Approaches in Drug Discovery, 2006, 15, 313.
Sawada, N.; Ishida, H.; Collins, B.E.; Schnaar, R.L.; Kiso, M. Carbohydr. Res. 1999, 316, 1.
Schwizer, D.; Gäthje, H.; Kelm, S.; Porro, M.; Schwardt, O.; Ernst, B. Bioorg. Med. Chem. 2006, 14, 4944.
Shelke, S.V.; Gao, G.-P.; Mesch, S.; Gäthje, H.; Kelm, S.; Schwardt, O.; Ernst, B. Bioorg. Med. Chem. 2007, 12, 4951-4965.
Sem, D.S.; Fragment-based Approaches in Drug Discovery, 2006, 8, 149.
Shuker, S. B., Hajduk, P. J., Meadows, R. P., Fesik, S V Science 1996, 274, 1531-1534.
Strenge, K.; Schauer, R.; Bovin, N.; Hasegawa, A.; Ishida, H.; Kiso, M.; Kelm, S. Eur. J. Biochem. 1998, 258, 677.
Vyas, A.A.; Blixt, O.; Paulson, J.C.; Schnaar, R.L. J. Biol. Chem. 2005, 280, 16305.
Vinson, M.; Strijbos, P.J.L.M.; Rowles, A.; Facci, L.; Moore, S.E.; Simmons, D.L.; Walsh, F.S. J. Biol. Chem. 2001, 276, 20280.
Yang, L.J.-S.; Zeller, C.B.; Shaper, N.L.; Kiso, M.; Hasegawa, A.; Shapiro, R.E.; Schnaar, R.L. Proc. Natl. Acad. Sci. USA 1996, 93, 814.

## Claims

1. A method for producing a new lead for a drug candidate comprising:
providing
(a) a target;
(b) an initial lead that binds to a first site at said target,
(c) at least one second-site ligand that binds to a second site at said target,
wherein the initial lead is substituted with a spin label to quench a NMR signal of the second-site ligand, when said second-site ligand is bound to the target,
identifying a second-site ligand whose NMR signal and/or paramagnetic relaxation is quenched or enhanced, respectively,
combining a homogenous pool of said initial lead, each initial lead in said pool being
substituted with a first linker comprising a first functional group, with a homogenous pool of said identified second-site ligand, each identified second-site ligand being substituted with a second linker comprising a second functional group,
wherein said first and second linker form a covalent linkage between said initial lead and said second-site ligand to produce a new lead for a drug candidate if
(1) the initial lead and said second-site ligand are bound to the target, and
(2) said first and second functional groups are oriented so that they react with each
other, and
identifying the lead for a drug candidate wherein the initial lead is covalently linked to said second-site ligand.

2. The method of claim 1, wherein the target has a structure that is unknown.

3. The method of claim 1 or 2, wherein the spin label is a TEMPO-derivate.

4. The method of any of the preceding claims, wherein the second-site ligand binds to the target proximate of the initial lead.

5. The method of claim 4, wherein the second-site ligand binds to the target in a distance to the initial lead of less than 30 Å.

6. The method of claim 4, wherein the second-site ligand binds to the target in a distance to the initial lead of less than 25 Å.

7. The method of claim 4, wherein the second-site ligand binds to the target in a distance to the initial lead of less than 20 Å.

8. The method of any of the preceding claims, wherein said first and second functional group react with each other in a chemical reaction to form a covalent linkage.

9. The method of claim 8, wherein said first functional group is an azide, said second functional group is an acetylene or *vice versa* and wherein said azide and said acetylene react with each other to form a triazole.

10. The method of any of the preceding claims, wherein said initial lead and/or said second-site ligand have micromolar (µM) binding affinities to the target.

11. The method of claim 10, wherein said initial lead and said second-site ligand form a new lead for a drug candidate having a binding affinity to the target exceeding a sum of the binding affinities of the initial lead and the second-site ligand.

12. The method of any of the preceding claims, wherein said at least one second-site ligand is part of a pool of compounds having a framework found in known drugs or having a set of properties found in drugs.

13. The method of claim 12, wherein
- the initial lead is substituted with a spin label and quenches a NMR signal of the second-site ligand when said second-site ligand is bound to the target, and
- said pool of compounds is subdivided into sub-pools, wherein the sub-pool is selected so that, in an NMR spectrum of the sub-pool at least one signal of each second-site ligand in said sub-pool remains distinguishable.

14. The method of any of the preceding claims, wherein said initial lead is selected from a pool of compounds having a framework found in known drugs or having a set of properties found in drugs.

15. The new lead for a drug candidate comprising the following compound:

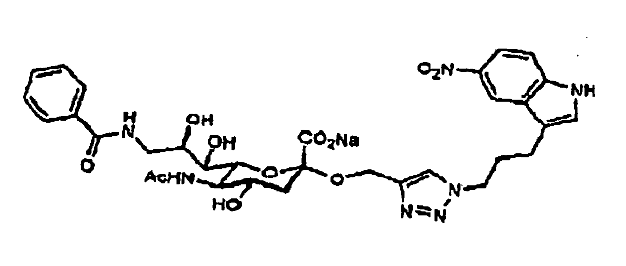

## Patentansprüche

1. Ein Verfahren zum Herstellen einer neuen Leitstruktur für einen Arzneistoffkandidaten, umfassend:
Bereitstellen
(a) eines Targets;
(b) einer anfänglichen Leitstruktur, die an eine erste Stelle an dem Target bindet,
(c) mindestens eines Liganden für eine zweite Stelle, der an eine zweite Stelle an dem Target bindet,
wobei die anfängliche Leitstruktur mit einer Spinmarkierung substituiert ist, um ein NMR-Signal des Liganden für eine zweite Stelle zu quenchen, wenn der Ligand für eine zweite Stelle an das Target gebunden wird,
Identifizieren eines Liganden für eine zweite Stelle, dessen NMR-Signal und/oder paramagnetische Relaxation gequencht bzw. verstärkt wird,
Kombinieren eines homogenen Pools der anfänglichen Leitstruktur, wobei jede anfängliche Leitstruktur in dem Pool mit einem ersten Linker substituiert ist, der eine erste funktionelle Gruppe umfasst, mit einem homogenen Pool des identifizierten Liganden für eine zweite Stelle, wobei jeder identifizierte Ligand für eine zweite Stelle mit einem zweiten Linker substituiert ist, der eine zweite funktionelle Gruppe umfasst,
wobei der erste und zweite Linker eine kovalente Verknüpfung zwischen der anfänglichen Leitstruktur und dem Liganden für eine zweite Stelle bilden, um eine neue Leitstruktur für einen Arzneistoffkandidaten zu erzeugen, falls
(1) die anfängliche Leitstruktur und der Ligand für eine zweite Stelle an das Target gebunden werden, und
(2) die erste und zweite funktionelle Gruppe derart ausgerichtet ist, dass sie miteinander reagieren, und
Identifizieren der Leitstruktur für einen Arzneistoffkandidaten, wobei die anfängliche Leitstruktur kovalent mit dem Liganden für eine zweite Stelle verknüpft ist.

2. Das Verfahren nach Anspruch 1, wobei das Target eine unbekannte Struktur aufweist.

3. Das Verfahren nach Anspruch 1 oder 2, wobei die Spinmarkierung ein TEMPO-Derivat ist.

4. Das Verfahren nach einem der vorstehenden Ansprüche, wobei der Ligand für eine zweite Stelle an das Target in der Nähe der anfänglichen Leitstruktur bindet.

5. Das Verfahren nach Anspruch 4, wobei der Ligand für eine zweite Stelle an das Target in einem Abstand zu der anfänglichen Leitstruktur von weniger als 30 Å bindet.

6. Das Verfahren nach Anspruch 4, wobei der Ligand für eine zweite Stelle an das Target in einem Abstand zu der anfänglichen Leitstruktur von weniger als 25 Å bindet.

7. Das Verfahren nach Anspruch 4, wobei der Ligand für eine zweite Stelle an das Target in einem Abstand zu der anfänglichen Leitstruktur von weniger als 20 Å bindet.

8. Das Verfahren nach einem der vorstehenden Ansprüche, wobei die erste und zweite funktionelle Gruppe in einer chemischen Reaktion unter Bildung einer kovalenten Verknüpfung miteinander reagieren.

9. Das Verfahren nach Anspruch 8, wobei die erste funktionelle Gruppe ein Azid ist, die zweite funktionelle Gruppe ein Acetylen ist, oder umgekehrt, und wobei das Azid und das Acetylen unter Bildung eines Triazols miteinander reagieren.

10. Das Verfahren nach einem der vorstehenden Ansprüche, wobei die anfängliche Leitstruktur und/oder der Ligand für eine zweite Stelle mikromolare (µM) Bindungsaffinitäten zu dem Target aufweisen.

11. Das Verfahren nach Anspruch 10, wobei die anfängliche Leitstruktur und der Ligand für eine zweite Stelle eine neue Leitstruktur für einen Arzneistoffkandidaten mit einer Bindungsaffinität zu dem Target bilden, die eine Summe der Bindungsaffinitäten der anfänglichen Leitstruktur und des Liganden für eine zweite Stelle übersteigt.

12. Das Verfahren nach einem der vorstehenden Ansprüche, wobei der mindestens eine Ligand für eine zweite Stelle Teil eines Pools von Verbindungen mit einem Grundgerüst, das in bekannten Arzneistoffen gefunden wird, oder mit einem Satz von Eigenschaften ist, der in Arzneistoffen gefunden wird.

13. Das Verfahren nach Anspruch 12, wobei
- die anfängliche Leitstruktur mit einer Spinmarkierung substituiert ist und ein NMR-Signal des Liganden für eine zweite Stelle quencht, wenn der Ligand für eine zweite Stelle an das Target gebunden wird, und
- der Pool von Verbindungen in Teilpools unterteilt ist, wobei der Teilpool derart ausgewählt ist, dass in einem NMR-Spektrum des Teilpools mindestens ein Signal von jedem Liganden für eine zweite Stelle in der Teilpool unterscheidbar bleibt.

14. Das Verfahren nach einem der vorstehenden Ansprüche, wobei die anfängliche Leitstruktur aus einem Pool von Verbindungen mit einem Grundgerüst, das in bekannten Arzneistoffen gefunden wird, oder mit einem Satz von Eigenschaften, der in Arzneistoffen gefunden wird, ausgewählt ist.

15. Die neue Leitstruktur für einen Arzneistoffkandidaten, umfassend die folgende Verbindung:

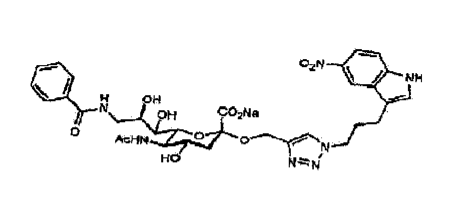

## Revendications

1. - Procédé de production d'une nouvelle tête de série pour un médicament candidat comprenant :
- se procurer
(a) une cible ;
(b) une tête de série initiale qui se lie à un premier site à ladite cible ;
(c) au moins un ligand de second site qui se lie à un second site à ladite cible,
la tête de série initiale étant substituée par un marqueur de spin pour désactiver un signal RMN du ligand de second site, lorsque ledit ligand de second site est lié à la cible ;
- identifier un ligand de second site dont le signal RMN et/ou la relaxation paramagnétique est respectivement désactivé ou amplifié,
- combiner un pool homogène de ladite tête de série initiale, chaque tête de série initiale dans ledit pool étant substituée par un premier lieur comprenant un premier groupe fonctionnel, avec un pool homogène dudit ligand de second site identifié, chaque ligand de second site identifié étant substitué par un second lieur comprenant un second groupe fonctionnel,
lesdits premier et second lieurs formant une liaison covalente entre ladite tête de série initiale et ledit ligand de second site pour produire une nouvelle tête de série pour un médicament candidat si
(1) la tête de série initiale et ledit ligand de second site sont liés à la cible, et
(2) lesdits premier et second groupes fonctionnels sont orientés de telle sorte qu'ils réagissent l'un avec l'autre, et
- identifier la tête de série pour un médicament candidat dans lequel la tête de série initial est liée de façon covalente audit ligand de second site.

2. - Procédé selon la revendication 1, dans lequel la cible a une structure qui est inconnue.

3. - Procédé selon l'une des revendications 1 ou 2, dans lequel le marqueur de spin est un dérivé de TEMPO.

4. - Procédé selon l'une quelconque des revendications précédentes, dans lequel le ligand de second site se lie à la cible à proximité de la tête de série initiale.

5. - Procédé selon la revendication 4, dans lequel le ligand de second site se lie à la cible dans une distance par rapport à la tête de série initiale de moins de 30 Å.

6. - Procédé selon la revendication 4, dans lequel le ligand de second site se lie à la cible dans une distance par rapport à la tête de série initiale de moins de 25 Å.

7. - Procédé selon la revendication 4, dans lequel le ligand de second site se lie à la cible dans une distance par rapport à la tête de série initiale de moins de 20 Å.

8. - Procédé selon l'une quelconque des revendications précédentes, dans lequel lesdits premier et second groupes fonctionnels réagissent l'un avec l'autre dans une réaction chimique pour former une liaison covalent.

9. - Procédé selon la revendication 8, dans lequel ledit premier groupe fonctionnel est un azide, ledit second groupe fonctionnel est un acétylène ou réciproquement et dans lequel ledit azide et ledit acétylène réagissent l'un avec l'autre pour former un triazole.

10. - Procédé selon l'une quelconque des revendications précédentes, dans lequel ladite tête de série initiale et/ou ledit ligand de second site ont des affinités de liaison micromolaire (µM) par rapport à la cible.

11. - Procédé selon la revendication 10, dans lequel ladite tête de série initiale et ledit ligand de second site forment une nouvelle tête de série pour un médicament candidat ayant une affinité de liaison par rapport à la cible dépassant une sommé des affinités de liaison de la tête de série initiale et du ligand de second site.

12. - Procédé selon l'une quelconque des revendications précédentes, dans lequel ledit au moins un ligand de second site fait partie d'un pool de composés ayant un squelette trouvé dans des médicaments connu ou ayant un ensemble de propriétés trouvé dans des médicaments.

13. - Procédé selon la revendication 12, dans lequel :
- la tête de série initiale est substituée par un marqueur de spin et désactive un signal RMN du ligand de second site lorsque ledit ligand de second site est lié à la cible ; et
- ledit pool de composés est subdivisé en sous-pools, le sous-pool étant choisi de telle sorte que, dans un spectre RMN du sous-pool, au moins un signal de chaque ligand de second site dans ledit sous-pool reste distinguable.

14. - Procède selon l'une quelconque des revendications précédentes, dans lequel ladite tête de série initiale est choisie parmi un pool de composés ayant un squelette trouvé dans de médicaments connus ou ayant un ensemble de propriétés trouvé dans des médicaments.

15. - Nouvelle tête de série pour un médicament candidat comprenant le composé suivant :
